# EUROPÄISCHE PATENTSCHRIFT

(11) **EP 1 304 336 B1**
(45) Veröffentlichungstag und Bekanntmachung des Hinweises auf die Patenterteilung: **25.04.2007**
(21) Anmeldenummer: 02022825.0
(22) Anmeldetag: 12.10.2002
(51) Int. Cl.: C07K 16/18, G01N 33/68, G01N 33/577, G01N 33/50, G01N 33/543, C07K 14/47

(54) **Antikörper zum spezifischen Nachweis von pathogenen Prionen humanen Ursprungs und damit durchgeführte Nachweisverfahren**
Antibodies for specific detection of pathogenic prions of human origin and detection methods using them
Anticorps pour la détection spécifique de prions pathogènes d'origine humaine et procédés de détection les utilisants

(30) Priorität: 19.10.2001 DE 10152677
(43) Veröffentlichungstag der Anmeldung: 23.04.2003
(73) Patentinhaber: CSL Behring GmbH, 35041 Marburg (DE)
(72) Erfinder: Vey, Martin, 35039 Marburg (DE); Lang, Wiegand, 35091 Coelbe (DE); Groener, Albrecht, 35041 Marburg (DE); Bellon, Anne, 35037 Marburg (DE)

(56) Entgegenhaltungen:
- WO-A-01/68695
- WO-A-97/37411
- US-A- 6 077 938
- HARMEYER S ET AL: "Synthetic peptide vaccines yield monoclonal antibodies to cellular and pathological prion proteins of ruminants" JOURNAL OF GENERAL VIROLOGY, SOCIETY FOR GENERAL MICROBIOLOGY, READING, GB, Bd. 79, 1998, Seiten 937-945, XP002178942 ISSN: 0022-1317
- KRASEMANN S ET AL: "Generation of monoclonal antibodies against prion proteins with an unconventional nucleic acid-based immunization strategy" JOURNAL OF BIOTECHNOLOGY, ELSEVIER SCIENCE PUBLISHERS, AMSTERDAM, NL, Bd. 73, Nr. 2-3, 20. August 1999 (1999-08-20), Seiten 119-129, XP004180175 ISSN: 0168-1656
- MURAMOTO TAMAKI ET AL: "Analyses of Gerstmann-Straussler syndrome with 102Leu219Lys using monoclonal antibodies that specifically detect human prion protein with 219Glu." NEUROSCIENCE LETTERS, Bd. 288, Nr. 3, 21. Juli 2000 (2000-07-21), Seiten 179-182, XP002253931 ISSN: 0304-3940
- KRASEMANN S ET AL: "Induction of antibodies against human prion proteins (PrP) by DNA-mediated immunization of PrPmice" JOURNAL OF IMMUNOLOGICAL METHODS, ELSEVIER SCIENCE PUBLISHERS B.V.,AMSTERDAM, NL, Bd. 199, Nr. 2, 15. Dezember 1996 (1996-12-15), Seiten 109-118, XP004071808 ISSN: 0022-1759
- KORTH C ET AL: "PRION (PRPSC)-SPECIFIC EPITOPE DEFINED BY A MONOCLONAL ANTIBODY" NATURE, MACMILLAN JOURNALS LTD. LONDON, GB, Bd. 390, Nr. 6655, 6. November 1997 (1997-11-06), Seiten 74-77, XP002069611 ISSN: 0028-0836
- MCRAE J L ET AL: "Human factor H-related protein 5 (FHR-5)" JOURNAL OF BIOLOGICAL CHEMISTRY, AMERICAN SOCIETY OF BIOLOGICAL CHEMISTS, BALTIMORE, MD, US, Bd. 276, Nr. 9, 2. März 2001 (2001-03-02), Seiten 6747-6754, XP002183722 ISSN: 0021-9258
- DATABASE BIOSIS [Online] BIOSCIENCES INFORMATION SERVICE, PHILADELPHIA, PA, US; 1996 RYU CHUN JEIH ET AL: "A humanized antibody with specificity for hepatitis B surface antigen" Database accession no. PREV199799456436 XP002264535 & HUMAN ANTIBODIES AND HYBRIDOMAS, Bd. 7, Nr. 3, 1996, Seiten 113-122, ISSN: 0956-960X
- DATABASE MEDLINE [Online] US NATIONAL LIBRARY OF MEDICINE (NLM), BETHESDA, MD, US; 1994 HELFRICH W ET AL: "Epitope mapping of SCLC-cluster-2 MAbs and generation of antibodies directed against new EGP-2 epitopes." Database accession no. NLM7515032 XP002264536 & INTERNATIONAL JOURNAL OF CANCER. SUPPLEMENT = JOURNAL INTERNATIONAL DU CANCER. SUPPLEMENT. UNITED STATES 1994, Bd. 8, 1994, Seiten 64-69, ISSN: 0898-6924
- DATABASE BIOSIS [Online] BIOSCIENCES INFORMATION SERVICE, PHILADELPHIA, PA, US; 1993 LIAO JIAN ET AL: "Monoclonal antibodies against brain acetylcholinesterases which recognize the subunits bearing the hydrophobic anchor" Database accession no. PREV199396090695 XP002264537 & EUROPEAN JOURNAL OF BIOCHEMISTRY, Bd. 215, Nr. 2, 1993, Seiten 333-340, ISSN: 0014-2956
- BRODNICKI T C ET AL: "reactivity and epitope mapping of single-chain T cell rceptors with monoclonal antibodies" MOLECULAR IMMUNOLOGY, ELMSFORD, NY, US, Bd. 33, Nr. 3, März 1996 (1996-03), Seiten 253-263, XP002212853 ISSN: 0161-5890

## Beschreibung

Die Erfindung betrifft Antikörper, die spezifisch an Prionen humanen Ursprungs binden sowie ein Verfahren zum Nachweis von pathogenen Prionen, insbesondere Erregern der spongiformen Enzephalopathie.

Prionerkrankungen wie z. B. die Creutzfeldt-Jakob-Erkrankung (CJD) können sowohl durch vererbte genetische Defekte entstehen als auch über noch nicht vollständig verstandene Infektionswege erworben werden. Darüber hinaus treten sie auch als spontane, sogenannte sporadische Formen auf, für die eine somatische Mutation im Gen für das Prionprotein postuliert wird (Prusiner SB., 1998) latrogene Infektionswege entstehen zum Beispiel durch Behandlung mit prionen-verseuchten Wachstumshormonen, Geschlechtshormonen oder Hornhautund Hirnhauttransplantaten. Auch die Verwendung von nicht ausreichend sterilisiertem chirurgischem Material stellt eine mögliche Infektionsquelle dar.

Die 33 bis 35 kD großen Prionproteine (abgekürzt PrP) kommen in einer natürlichen physiologischen Isoform (PrP^{c}) sowie in einer pathologisch infektiösen Isoform (PrP^{Sc}) vor, wobei die infektiöse Isoform durch eine Umfaltung der Sekundär- und Tertiärstruktur aus der nichtinfektiösen physiologischen Form entsteht. Das PrP^{Sc} ist höchstwahrscheinlich die einzige stoffliche Komponente der Prionen, die für die Transmission und die Pathogenese der Prionenerkrankungen verantwortlich ist (Prusiner SB., 1998)

Aus Prusiner, Cell (Prusiner SB, 1984) sowie Biochemistry (Prusiner SB, 1982) ist es bereits bekannt, dass Prionproteine einer partiellen Proteolyse zugänglich sind. In der Folgezeit hat es sich gezeigt, dass PrP^{c} nahezu vollständig einer Proteolyse zugänglich ist, wohingegen PrP^{Sc} sich lediglich bis zu einer Größe von 27 bis 30 kD abbauen lässt. Diese einer weiteren Proteolyse nicht zugängliche Proteinform wird als ein gegen Protease beständiger Kern, kurz PrP²⁷⁻³⁰, bezeichnet. Sie entsteht durch Abbau von ca. 67 Aminosäuren am NH₂-Terminus und besteht selbst aus ca. 141 Aminosäuren.

Es sind auch bereits Verfahren zum Nachweis der pathologischen Prion-Isoformen beschrieben worden. So beschreiben beispielsweise Barry und Prusiner (Barry RA, 1986) einen Westernblot-Test unter Verwendung eines monoklonalen Anti-Prionprotein Antikörpers (MAK) 13A5. Dieser Hamster-PrP spezifische MAK wurde in Mäusen gewonnen, die mit gereinigtem, denaturiertem PrP²⁷⁻³⁰ isoliert aus Scrapie-infizierten Hamstern, immunisiert worden waren.

Es sind auch bereits andere Antikörper beschrieben, die wie der MAK 13A5 sowohl gegen PrP^{C} als auch gegen PrP^{Sc}, sofern dieses denaturiert vorliegt, gerichtet sind (US Patent 4806627). Desweiteren sind Immunisierungen mit rekombinanten Prionproteinen, welche in Bakterien exprimiert worden waren, durchgeführt worden, so beschrieben in Zanusso et al. (Zanusso G, 1998). Ebenso gelang die Herstellung monoklonaler Antikörper mittels Peptidimmunisierung, so beschrieben in Harmeyer et al.,(Harmeyer S, 1998) und mittels Nukleinsäure-lmmunisierung, wie in Krasemann(Krasemann S, 1999) erläutert.

Eine weitere Anwendung dieser Antikörper neben dem Westernblot wurde in dem US Patent 4806627, Wisniewski et al. erwähnt, nämlich ein so genannter ELISA (enzyme-linked immunosorbent assay). In diesem ELISA wurden Prionen, welche auf einer Mikrotiter-Platte fixiert waren, von dem MAK 3F4 gebunden und dieser dann mittels eines Zweitantikörpers, der über ein an ihm gekoppeltes Enzym eine Farbstoffreaktion katalysiert, detektiert.

In all diesen Nachweisverfahren wird die Probe mit dem Enzym Proteinase K vorbehandelt, um in der Probe befindliches, normales Prionprotein zu entfernen und somit den alleinigen Nachweis des Protease-resistenten, pathogenen Prionproteins zu gewährleisten, da ja die Antikörper auch das normale Prionprotein mit hoher Affinität binden können.

Die zuvor beschriebenen Methoden mittels Auftrennen durch Elektrophorese und Immobilisierung an Membranen, insbesondere Nitrocellulose-Membranen und anschließender Bestimmung mittels Anti-PrP-Antiserum (Western blot), sind jedoch als Methode für Reihenuntersuchungen aufgrund der notwendigen Arbeits- und Zeitintensität wenig geeignet. Aufgrund der Bedrohung der Bevölkerung durch eine mögliche Übertragung von spongiformen Enzephalopathien besteht daher ein großer Bedarf an einem schnellen Nachweisverfahren für Prionen, beispielsweise in der Human- und Veterinärdiagnostik, wobei in entnommenen Körperflüssigkeiten und Gewebeproben die pathologische Prion-lsoform qualitativ und quantitativ nachgewiesen werden kann.

Schließlich ist auch schon aus der internationalen Patentanmeldung WO 98/37411 ein Nachweisverfahren bekannt, mit dem der Nachweis der pathogenen Konformation des Prionproteins in einer Probe möglich ist. Die Probe wird dabei in zwei Portionen aufgeteilt und die erste Portion an einen festen Träger gebunden und dann mit einem markierten Antikörper kontaktiert. Dieser Antikörper bindet an die nicht-pathogene Form des Prionproteins mit höherer Affinität als an die nicht denaturierte, pathogene Form des Proteins. Die zweite Portion der Probe wird dann einer Behandlung unterzogen, durch die sich die Konformation des pathogenen Prionproteins ändert und dadurch sich die Zugänglichkeit und damit die Affinität für den markierten Antikörper drastisch erhöht. Die so behandelte zweite Probe wird dann mit einem zweiten Träger in Berührung gebracht und mit dem markierten Antikörper umgesetzt. Dann werden die Mengen des in der ersten und in der zweiten Portion der Probe gebundenen markierten Antikörpers gemessen und miteinander verglichen. Ein Unterschied zwischen beiden Messergebnissen zeigt an, ob die pathogene Form des Prionproteins in der Probe vorhanden war, die Größenordnung des Unterschiedes korreliert mit der Menge an der pathogenen Form des Prionproteins. Dieses Nachweisverfahren wird konformationsabhängiger Immunoassay genannt, im Englischen mit der Abkürzung CDI für Conformation-Dependent Immunoassay versehen. Die Empfindlichkeit des CDIs lässt sich erhöhen, wenn die Probe einer Vorbehandlung mit einem proteolytischen Enzym, beispielsweise der Proteinase K oder der Dispase unterworfen wird, da die Behandlung mit Proteasen PrP^{c} und nicht-relevante Proteine in der Probe zerstört werden und das Protease-resistante PrP²⁷⁻³⁰ in der Probe übrig bleibt.

Die Untersuchung von humanem Blutplasma auf Anwesenheit des pathogenen Prionproteins erfordert sehr empfindliche und spezifische Nachweissysteme, die sich auch für die Automatisierung eignen. Erschwert wird der Nachweis dadurch, dass die physiologischen Grundlagen für die pathologische Wirkung von Prionen noch nicht bekannt sind. Außerdem gibt es bisher noch keine diagnostischen Reagenzien, die direkt zwischen der pathologischen Isoform PrP^{Sc} und der normalen Isoform PrP^{c} unterscheiden, die meistens in großem Überschuss vorhanden ist und mit allen Antikörpern, die bisher zum Prionennachweis eingesetzt werden, kreuzreagiert.

Allen bisher bekannten Nachweisverfahren für pathogene Prionproteine ist gemeinsam, dass nicht eindeutig unterschieden werden kann, ob es sich um Prionproteine humanen Ursprungs oder um Prionproteine handelt, die aus anderen Spezies stammen. Der sehr weit verbreitete monoklonale Antikörper (MAk) 3F4, der für die Diagnostik humaner Prionenerkrankungen eingesetzt wird, reagiert auch mit den Prionproteinen anderer Säugetierspezies, so z.B. mit dem des Hamsters. Eine Unterscheidungsmöglichkeit wäre sehr wichtig, um zu erkennen, ob das pathogene Prionprotein in den menschlichen Organismus gelangt ist oder ob die pathogenen Prionen erst im menschlichen Organismus entstanden sind. Die Übertragung tierischer Prionen auf den Menschen könnte zum einen durch Exposition am Arbeitsplatz, z.B. in einem Labor oder Tierställen, in denen mit Prionen umgegangen wird, zum anderen aber auch durch Verzehr von Prionenkontaminierten Lebensmitteln oder evtl. sogar durch die Verwendung kontaminierter kosmetischer oder pharmazeutischer Produkte erfolgen. Die genaue Kenntnis der Infektionsquelle könnte wichtige Erkenntnisse über die Verbreitung von Prionenerkrankungen, z.B. der BSE, deren Verbreitung innerhalb der menschlichen Population sowie innerhalb eines Einzelorganismus bringen und wirksame Schutzmaßnahmen ermöglichen.

Höchstspezifische Nachweisverfahren für pathogene Prionen humanen Ursprungs sind bisher daran gescheitert, dass mit den bisher bekannten Antikörpern kein Epitop selektiv erkannt werden konnte, das nur bei Prionen humanen Ursprungs und nicht gleichzeitig auch bei Prionen tierischen Ursprungs vorkommt.

Überraschenderweise wurde nun gefunden, dass es möglich ist, Antikörper, insbesondere auch monoklonale Antikörper, aufzufinden, die ein für ein humanes Prionprotein charakteristisches Epitop erkennen, jedoch mit einem Prionprotein tierischen Ursprungs nicht reagieren. Die Erfindung umfaßt auch ein wie in Anspruch 1 definiertes konformationelles Epitop, welches für humanes Prionprotein spezifisch ist. Diese selektive Erkennung des humanen Prion Proteins wurde vor allem im Western Blot Verfahren deutlich. Beispiele für derartige Antikörper sind die von den Hybridoma-Zelllinien DSM ACC 2522, DSM ACC 2523 und DSM ACC 2524 gebildeten monoklonalen Antikörper. Diese monoklonalen Antikörper zeigen keine Kreuzreaktivität mit dem PrP der afrikanischen grünen Meerkatze oder mit bovinem, Hamster-, Ratten- oder Maus-PrP, welche in SDS-Polyacrylamid-Gelen aufgetrennt, auf Nylonmembranen transferiert und mit den Antikörpern versetzt wurden.

Mit Hilfe dieser neuen monoklonalen Antikörper können nun erstmals Verfahren zum hochspezifischen Nachweis von Prionproteinen humanen Ursprungs entwickelt werden, zum Beispiel auf der Basis des oben beschrieben Westernblot-Verfahrens.

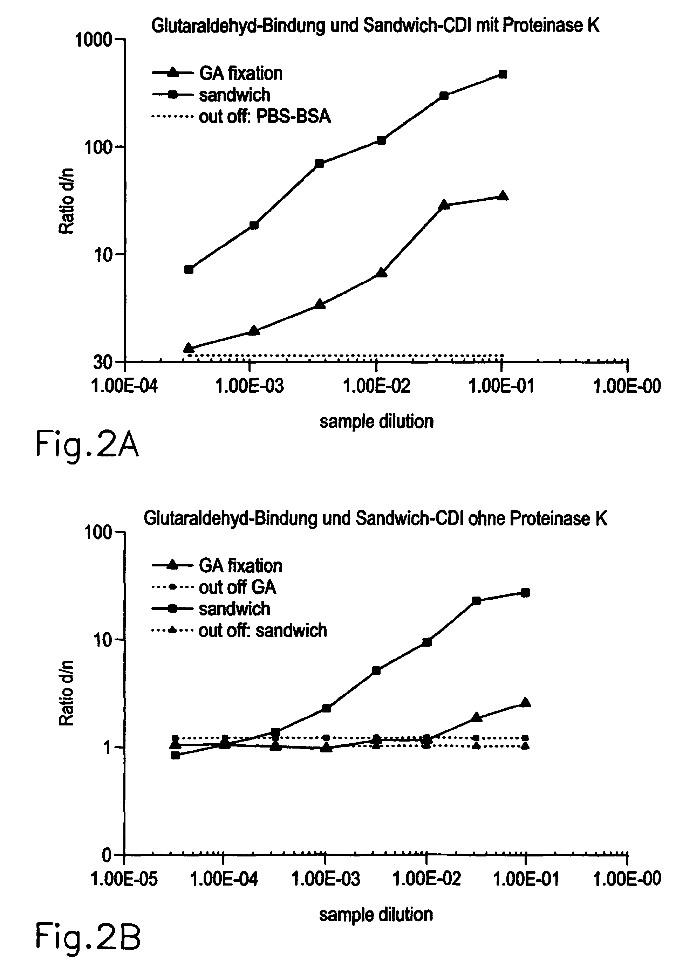

Von besonderem Interesse ist außerdem das konformationsabhängige Immunoassay-Verfahren CDI zum Nachweis von pathogenen Prionproteinen in einer Probe einer Körperflüssigkeit oder eines verflüssigten Gewebes. Beim bisher verwendeten CDI erfolgte das Fixieren der zu untersuchenden Probe auf einem festen Träger mittels chemischer Quervernetzung mit Glutaraldehyd. Dadurch wurden die in der Probe enthaltenen Prionproteine nicht selektiv aus der Probe angereichert, sondern mit vielen anderen, nicht relevanten Proteinen auf der Trägersubstanz mit angeheftet. Dies bedeutet einen Empfindlichkeitsverlust, da viele Prionproteine in der Probe gar nicht auf dem Trägermaterial gebunden werden. Dieser Störeffekt kommt vor allem in Proben mit hohem Proteingehalt, z.B. Plasma, zum Tragen. Desweiteren wird hierdurch eine weitere Verwendung des CDIs, nämlich die Bestimmung des Prionengehaltes in Proben ohne Proteinase-Vorbehandlung, stark eingeschränkt, da ein Verzicht auf Proteinase-Behandlung diesen Störeffekt der nicht relevanten Proteine in der Probe noch erhöht. Die monoklonalen, Human-PrP spezifischen Antikörper, die von den hinterlegten Zellinien DSM ACC 2522, DSM ACC 2523 und DSM ACC 2524 produziert werden, heben diesen Störeffekt auf, da sie z.B. als Fängerantikörper auf das Trägermaterial aufgetragen werden können und dann die in der Probe befindlichen humanen Prionproteine selektiv auf dem Trägermaterial binden. Somit werden wesentlich mehr Prionproteine aus der Probe auf dem Trägermaterial gebunden und gleichzeitig die Empfindlichkeit des Tests 10-30-fach erhöht (Beispiel 2, Abb. 2 A). Somit ist auch ein Störeffekt nicht relevanter Proteine unerheblich und auch bei einem Verzicht auf eine Protease-Vorbehandlung der Proben wird eine hohe Testsensitivität erreicht (Beispiel 2, Abb. 2 B): hierbei bewirkt die Verwendung des Fängerantikörpers sogar eine 100- bis 300-fache Erhöhung der Testsensitivität im Vergleich mit der Glutaraldehyd-Vernetzungsmethode. Diese Konfiguration mit dem auf dem Träger aufgebrachten Fängerantikörper ist zur Vereinfachung als Sandwich-Konfiguration bezeichnet (Abbildung 2). Das verbesserte CDI-Nachweisverfahren umfasst folgende Stufen:
a) Versetzen einer ersten Portion der Probe mit einem der vorstehend genannten monoklonalen Antikörper, der an einen festen Träger fixiert ist und eine höhere Affinität für die erste Prionprotein-Konformation aufweist, sowie die Bestimmung dieser ersten Konzentration;
b) Behandlung der zweiten Portion der Probe zur Erhöhung der Bindungsaffinität der zweiten Konformation des Prionproteins an dem monoklonalen Antikörper;
c) Versetzen der behandelten zweiten Portion der zu untersuchenden Probe mit dem monoklonalen Antikörper, um die zweite Konzentration zu ermitteln;
d) Vergleichen der ersten Prionprotein-Konzentration mit der zweiten Prionprotein-Konzentration, um die Anwesenheit der pathogenen Prionprotein-Konformation zu ermitteln.
   Für dieses Nachweisverfahren sind insbesondere monoklonale Antikörper geeignet, die von den Hybridoma-Zelllinien DSM ACC 2522, DSM ACC 2523 und DSM ACC 2524 gebildet werden.
   Ein wichtiger Schritt des Immunoassay-Nachweisverfahrens besteht in der Behandlung eines Teils der zu untersuchenden Probe unter physikalischen oder chemischen Bedingungen, die zu einer Konformationsänderung der pathologischen Form des pathogenen Prionproteins führt, durch die die Zugänglichkeit für den Antikörper gesteigert wird. Die Konformationsänderung kann durch Hitze, Druck oder Einwirkung chemischer Substanzen erfolgen. Es reicht aus, wenn wenigstens 2% des anwesenden pathogenen Prionproteins in eine Konformation überführt wird, die für den den monoklonalen Antikörper zugänglich ist.

Als Trägermaterial zur Bindung der Prionproteine eignen sich die üblicherweise eingesetzten Chromatographieharze, Agarose, Mikrotiterplatten, Nitrocellulosemembranen, Nylonmembranen oder auch magnetische Körner (magnetic beads). Die an diese Trägermaterialien gebundenen Prionproteine werden beispielsweise mit einem ELISA-Verfahren nachgewiesen, bei dem ein zweiter markierter Antikörper mit den fixierten Prionproteinen in Berührung gebracht wird. Als zweiter markierter monoklonaler Antikörper hat sich der monoklonale Antikörper 3F4 bewährt. Er kann mit einer radioaktiven Gruppe, einer enzymatischen Gruppe oder einer fluoreszierenden Gruppe markiert sein.

Der Vorteil des erfindungsgemäßen Immunoassay-Nachweisverfahrens besteht darin, dass die Empfindlichkeit des Prionprotein-Nachweises etwa 10- bis 300-fach höher ist als die Empfindlichkeit bekannter konformationsabhängiger Immunoassays, wie sie beispielsweise aus der internationalen Patentanmeldung WO 98/37411 bekannt sind. Das wird durch den Einsatz der erfindungsgemäßen monoklonalen Antikörper erreicht, mit denen eine selektive Anreicherung der Prionproteine aus der zu untersuchenden Probe möglich ist.

Die Zuverlässigkeit und Empfindlichkeit des erfindungsgemäßen Nachweisverfahrens konnte dadurch gezeigt werden, dass Gruppen von 10 und 100 Plasmaproben mit ganz geringen Mengen von Prionen versetzt wurden und in allen Fällen ein sicherer Nachweis der Prionen mit dem erfindungsgemäßen Immunosassay gelang. Dieser konformationsabhängige Immunoassay zeigt sowohl Prionen an, die als Verursacher der Creutzfeldt-Jakob-Krankheit (CJD) als auch der neuen Variante der CJD (vCJD), die u.a. durch das Auftreten von Prionen in lymphatischen Organen von Patienten gekennzeichnet ist, gelten. Im Gegensatz dazu wurde in allen Plasmaproben, denen keine Prionen zugesetzt waren, außer einem geringfügigen Hintergrundsignal keinerlei positive Reaktion gefunden. Diese Ergebnisse konnten sowohl mit hochgereinigten Prionproteinen als auch mit homogenisierten Gehirnproben von infizierten Menschen oder von transgenen Mäusen, welche humane Prionen vermehren, nachgewiesen werden. Selbst wenn die bei allen bisherigen Verfahren notwendige Vorbehandlung mit einer Proteinase nicht durchgeführt wird, kann mit dem erfindungsgemäßen Immunoassayverfahren immer noch die Anwesenheit von Prionproteinen mit großer Sicherheit festgestellt werden.

Ein besonderes Merkmal der erfindungsgemäßen monoklonalen Antikörper besteht darin, dass sie nur humane Prionproteine erkennen. Dies liegt wahrscheinlich daran, dass eine ganz bestimmte Aminosäuresequenz des humanen Prionproteins, die nicht bei anderen Säugern vorkommt, erkannt wird.
Hieraus ergeben sich neue Anwendungsmöglichkeiten des erfindungsgemäßen Immunoassays. Findet man nämlich im menschlichen Organismus ein pathologisches Prionprotein mit den erfindungsgemäßen Antikörpern, so ist eindeutig, dass dieses PrP^{Sc} erst im menschlichen Organismus entstanden ist. Findet man dagegen kein Signal mit diesen erfindungsgemäßen Antikörpern, wohl aber ein Signal mit dem 6H4-Antikörper (Prionics AG, Schweiz), dann muss dieses pathologische Prionprotein tierischer Herkunft sein. In diesem Fall ist also der untersuchte Mensch mit Prionen tierischer Herkunft inokuliert. Damit können Prionproteine unbekannter Herkunft einer tierischen oder menschlichen Quelle zugeordnet und damit die Verbreitungswege pathologischer Prionen erkannt werden.

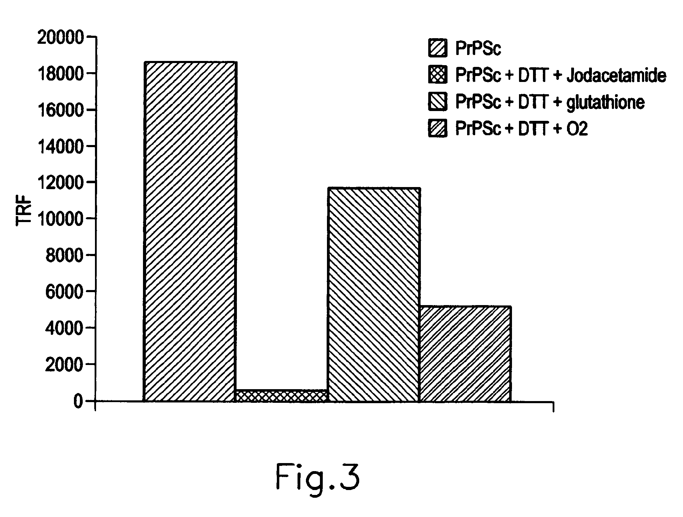

Die Bindungsstellen, Epitope, der erfindungsgemäßen monoklonalen Antikörper wurden ebenfalls charakterisiert. Das Epitop für die monoklonalen Antikörper DSM ACC 2522, DSM ACC 2523, DSM ACC 2524, wird maßgeblich von der einzigen Disulfidbrücke des Prionproteins bestimmt. Die Disulfidbrücke ist demnach entweder selbst Teil des Epitops und interagiert mit der Antigen-Bindungsstelle des monoklonalen Antikörpers, oder die Disulfidbrücke verbindet ansonsten voneinander entfernte Proteinabschnitte, die durch die Schwefelbrücke ein zusammengesetztes, konformationelles Epitop bilden, welches nur unter oxidierenden Bedingungen im Prionprotein vorhanden ist. Dies wird im Beispiel 4 und Abbildung 3 deutlich.

Die Antigenität von Proteinen lässt sich durch die Bindung von Antikörpern, die polyklonalen oder monoklonalen Ursprungs sind oder als single-chain Antikörper oder in Phage-Display-Systemen erzeugt wurden, charakterisieren. Diese Antikörper binden an bestimmte Aminosäure-Sequenzen der Proteine, sogenannten Epitopen. Diese Charakterisierung der Proteineigenschaften lässt sich mit dem Fachmann bekannten Methoden wie z.B. Western Blot, Bindung der Antikörper an Peptide, die anhand der Proteinsequenz (bzw. Nucleinsäuresequenz) des Proteins als sich in der Sequenz überlappende Peptide synthetisiert worden waren, ELISA mit an eine Oberfläche gebundenes Gesamtprotein, Bindung des Antigen/Antikörperkomplexes an Protein A oder Immunpräzipitation durchführen. Da Epitope entweder kontinuierlich (linear) oder diskontinuierlich (konformationell) sein können, kommen unterschiedliche Methoden zu deren Charakterisierung in Frage. Lineare Epitope bestehen aus hintereinandergereihten Atomen innerhalb desselben Proteinmoleküls, die räumliche Anordnung des Proteins (sekundäre, tertiäre und quartäre Struktur) hat deshalb keinen Einfluß auf das Epitop und die Bindung von Antikörpern an dieses Epitop. Die Behandlung von Proteinen mit denaturierenden Substanzen wie SDS oder chaotropen Substanzen hat deshalb keinen Einfluss auf die Antigen/Antikörper-Bindung. Diese Epitope lassen sich auch durch die Bindung der zu untersuchenden Antikörper an Peptidsequenzen des Gesamtproteins, die sich überlappen, in einem ELISA bestimmen. Binden die Antikörper an das Gesamtprotein, nachgewiesen z.B. in einem ELISA-System, einem DOT-Blot oder in Immunpräzipitationen, jedoch nicht nach Denaturierung des Proteins, liegt das Epitop als diskontinuierliches (oder konformationelles) Epitop vor, das aus Atomen des Proteins besteht, die nicht hintereinander angeordnet sind, jedoch auf demselben Proteinmolekül liegen und durch die räumliche Anordnung des Proteins in Nachbarschaft zueinander stehen. Diese räumliche Nachbarschaft kann auch bei verschiedenen Proteinen bzw. unterschiedlichen Domänen eines komplexen Proteins bestehen.

Überraschenderweise wurde bei der Charakterisierung der Bindungseigenschaften monoklonaler Antikörper, die von den Hybridoma-Zellinien DMS-ACC 2522, DMS-ACC 2523 und DMS-ACC 2524, gebildet werden, festgestellt, dass denaturiertes Protein im Westernblot mit dem MAk reagiert, was auf ein lineares Epitop hinweist; die Bindung des MAk an (überlappenden) Peptidsequenzen erfolgte jedoch nicht, was im Gegensatz zu dem ersten Versuchsergebnis auf ein diskontinuierliches Epitop hinweist. Bei der Überprüfung der Proteinsequenz wurde festgestellt, dass im Bereich der potentiellen Antikörperbindungsstellen eine Disulfidbrücke vorhanden ist. Es wurde nun untersucht, ob die Disulfidbindung zu einem konformationellen (diskontinuierlichen) Epitop führt.

Disulfidbrücken werden unter den üblichen, dem Fachmann bekannten proteindenaturierenden Bedingungen nicht aufgelöst, so dass im Westernblot (nach Auftrennung der Proteine hinsichtlich Molekulargewichts in einer SDS-Polyacrylamid-Gelelektrophorese und nachfolgendem Übertrag der Proteine aus dem Gel auf eine Membran), das Protein nach Inkubation der Membran in antikörperhaltiger, wassriger Lösung und anschließender Detektion mit markierten Zweitantikörpern, welche gegen den gebundenen ersten Antikörper gerichtet sind, nachgewiesen werden können. Wird jedoch die Disulfidbrücke unter reduzierenden Bedingungen aufgebrochen, ist das Epitop zerstört, so dass die Antikörper nicht mehr binden. Die Anwendung überlappender Peptide zur Charakterisierung von Epitopen, welche üblicherweise von Peptiden ohne Disulfidbrücken ausgeht, und zur Identifizierung der Epitope, welche sich im Westernblot nachweisen lassen verwendet wird, hat im Falle der Charakterisierung der Epitope der MAks DMS-ACC 2522, DMS-ACC 2523 und DMS-ACC 2524 keine Antikörperbindung nachweisen können, da die notwendige Disufidbrücke nicht in den Peptiden ausgebildet werden konnte und somit die für die Antikörperbindungsstelle notwendige Konformation nicht vorhanden war.

Die korrekte Ausbildung von Disulfidbrücken ist ein wichtiger Schritt in der Reifung von Proteinen und in den meisten Fällen ist die Ausprägung der Disulfidbrücken von entscheidender Bedeutung für die Stabilität und die Funktion der Proteine. Es ist bisher nicht mit einfachen Methoden die Disulfidbrücken selbst und deren korrekte Ausbildung nachzuweisen. Der überraschende Nachweis von Disulfidbrücken-verursachten Epitopen kann zur gezielten Charaktierisierung von Proteinen genutzt werden, indem Antikörper oder andere Hochaffinitätsliganden gegen Proteine mit Disulfidbrücken hergestellt und mit dem Fachmann bekannten Methoden hinsichtlich Bindung an Proteine mit Disulfidbrücken (unter reduzierten Bedingungen) und unter oxidierten Bedingungen (Disulfidbrücke gelöst) selektiert werden. Zur Herstellung solcher Antikörper werden Tiere mit Proteinen, welche Disulfidbrücken enthalten, immunisiert. Diese Proteine zur Immunisierung können entweder rekombinant in Bakterien-, Hefen-, Pflanzen-, Insekten-, Säugerzellen oder in vitro exprimiert und mit dem Fachmann bekannten Reinigungsmethoden isoliert werden oder direkt aus Geweben, Organen, Flüssigkeiten, Gemischen oder sonstigen Quellen ohne vorherige Überexpression gereinigt werden. Die Proteine können vor der Immunisierung reduziert oder aber unter Luftsauerstoffbedingungen, also oxidiert, zur Immunisierung verwendet werden. Ebenso können andere Hochaffinitätsliganden, z.B. so genannte "Single chain antibodies" oder Peptidliganden, in Liganden-Bibliotheken, z.B. sogenannten "Phage Display Libraries", identifiziert werden, indem
Die Information über das Vorhandensein oder das Fehlen von Disulfidbrücken ist von besonderer Bedeutung bei der industriellen Herstellung, Aufarbeitung, Veränderung und Reinigung von Proteinen zu Zwecken der therapeutischen, diagnostischen oder anderen Anwendungen. Mit Antikörpern oder ähnlichen Hochaffinitätsliganden, welche spezifisch Disulfidbrücken in Proteinen erkennen, können herkömmliche, einfache Verfahren, z.B. Westernblot etc., zum Nachweis dieser wichtigen Proteineigenschaft verwendet werden. So könnte ein solcher Antikörper z.B. mit geringem Aufwand Auskunft über den Zustand von Proteinen nach bestimmten Modifikationen zur Inaktivierung von kontaminierenden Erregern (Smales, 2002) oder nach rekombinanter Expression in Fremdorgansimen geben.

Die Durchführung des erfindungsgemäßen Nachweises wird durch die nachfolgenden Beispiele veranschaulicht:

### Beispiele:

### 1. Westernblot-Nachweis von PrP^{c} verschiedener Spezies mittels der monoklonalen Antikörper aus de Hybridoma-Zelllinien DSM ACC 2522, DSM ACC 2523, DSM ACC 2524

Zum Nachweis von PrP^{c} von unterschiedlichen Spezies wurden gleiche Gewichtsanteile von Gehirn einer afrikanischen grünen Meerkatze, eines Rinds, eines syrischen Goldhamsters, einer Ratte und einer transgenen Maus, die humanes PrP exprimiert, in einer phosphatgepufferten Salzlösung (PBS) mit einer Ultra Turrax Gewebemühle homogenisiert und eine Endkonzentration von 10% (Gewicht/Volumen) hergestellt. Große Gewebstrümmerteile wurde durch Zentrifugieren über einen Zeitraum von 15 Minuten bei 500xg bei Raumtemperatur entfernt. Zu den Überständen wurden gleiche Volumina von PBS, die 4% Sarkosin enthielten, hinzugefügt. Zur Proteintrennung wurden diese Lysate mit der Pufferlösung für die SDS-Gelelektrophorese gemischt und 10 Minuten bei 100°C gekocht. 20 µl des Gehirnlysates jeder Spezies wurden dann auf ein 10%-iges SDS-Polyacrylamidgel (Novex) geladen und die Proteine durch Elektrophorese über einen Zeitraum von 45 Minuten bei 150 Volt getrennt. Die Proteine wurden dann durch einen Elektrotransfer auf Nylonmembranen (Millipore) übertragen. Die Membranen wurden dann mit TBST (Tris-buffered saline/0,1% Tween 20 (Sigma)), welches 1% Rinderserum-albumin (BSA, Merck) enthält, 60 Minuten bei Raumtemperatur blockiert und entweder mit dem monoklonalen Antikörper 6H4 (erhältlich bei Prionics AG, Schweiz), verdünnt 1:5.000 in TBST, oder mit dem monoklonalen Antikörper der Hybridoma-Zelllinie DSM ACC 2522, DSM ACC 2523 oder DSM ACC 2524, verdünnt 1:1.000 in TBST, oder mit TBST eine Stunde bei Raumtemperatur unter Schütteln inkubiert. Nach dreimaligem Waschen mit TBST wurden die Membranen mit einem Ziegen-anti-Maus IgG-Antikörper konjugiert mit alkalischer Phosphatse(Amersham), die in TBST 1:5.000 verdünnt war, eine Stunde bei Raumtemperatur unter Schütteln inkubiert. Nach fünfmaligem Waschen mit TBST wurden die Membranen mit dem Nachweisreagenz (Amersham) inkubiert und die Proteinbanden nachgewiesen.

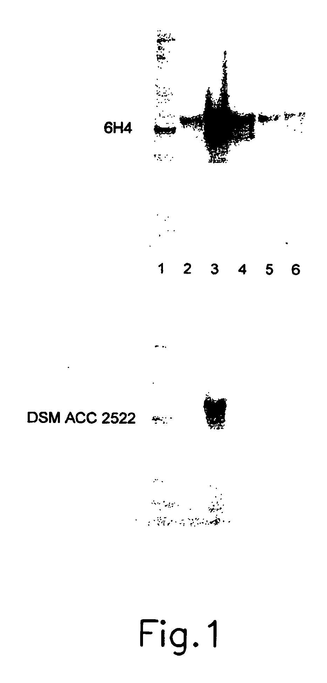

Die aus der Zelllinie DSM ACC 2522, DSM ACC 2523 oder DSM ACG 2524 gewonnenen monoklonalen Antikörper besitzen keine Kreuzreaktivität mit Rinder-, Hamster-, Ratten- oder dem PrP der afrikanischen grünen Meerkatze wohl aber eine eindeutige Bindung an humanes Prionprotein, während MAk 3F4 Hamster und humane Prionproteine erkennt (Fig. 1).

### 2. Verbessertes, konformationsabhängiges Immunoassayverfahren mit dem monoklonalen Antikörper aus der Hybridoma-Zelllinie DSM ACC 2522, DSM ACC 2523 oder DSM ACC 2524 als Bindungsreagenz: Sandwich-CDI

Das Gehirn einer transgenen Maus, die humanes PrP^{c} exprimiert und mit der sporadischen Form der Creutzfeldt-Jakob-Krankheit (sCJD) infiziert war, wurde aus einer euthanasierten Maus beim ersten Anzeichen der Prionenerkrankung isoliert, d.h. etwa 150 Tage nach der intrazerebralen Infektion mit Gehirnhomogenat von einem an CJD verstorbenen Patienten. Das Gehirn wurde nach den Verfahren zur Lysatherstellung gemäß Beispiel 1 lysiert. Das Gehirnlysat wurde mit PBS, welches 2% Sarcosyl (Gewicht/Volumen) und 4% BSA (Gewicht/Volumen) in 0,5 log₁₀ Stufen verdünnt. Die Proben wurden dann mit Proteinase K (PK; Roche) bei einer Endkonzentration von 250 µg/ml eine Stunde bei 37°C behandelt. Die Verdauungen wurden durch Zugabe der Proteinaseinhibitoren PMSF (=Phenylmethylsulfonylfluorid; Roche), Aprotinin (Sigma) und Leupeptin (Sigma) gestoppt, wobei die Endkonzentration jeweils 20 µg/ml betrug. Phosphowolframsäure wurde bis zu einer Endkonzentration von 0,3% und Magnesiumchlorid bis zu einer Endkonzentration von 2,72 mM zugegeben. Die Proben wurden dann 16 Stunden lang bei 37°C inkubiert und nachfolgend bei 14.000 rpm über einen Zeitraum von 30 Minuten bei Raumtemperatur zentrifugiert. Die Überstände wurden entfernt und die Pellets in 50 µl H₂O resuspendiert, welches die Proteinaseinhibitoren Leupeptin und Aprotinin in einer Menge von 0,1 µg/ml enthielt. Die Proben wurden aufgeteilt und eine Hälfte durch Zugabe von 25 µl von 8 M Guanidinium-hydrochlorid (Gdn-HCI; Merck) und Erhitzen auf 80°C in einem Zeitraum von 5 Minuten denaturiert. Nach Abkühlen auf Raumtemperatur wurden 950 µl H₂O, enthaltend die Proteinaseinhibitoren Leupeptin und Aprotinin in einer Menge von 0,1 µg/ml hinzugegeben. Die unbehandelten Hälften der Proben wurden mit 975 µl H₂O, das die gleiche Proteinaseinhibitoren und 0,205 M Gdn-HCI enthielt, verdünnt. Je 3 Aliquots der verdünnten denaturierten und nichtdenaturierten (nativen) Proben wurden auf Testplatten mit 96 Vertiefungen (200 µl/Vertiefung) übertragen, dessen Vertiefungen zuvor entweder mit PBS, das 0,2% Glutaraldehyd enthielt, 2 Stunden bei Raumtemperatur reaktiviert worden waren oder mit dem monoklonalen Antikörper aus DSM ACC 2522, DSM ACC 2523 oder DSM ACC 2524 beschichtet waren, der in PBS bis zu einer Endkonzentration von 10 µg/ml verdünnt worden war. Die Proben wurden auf den präaktivierten Platten 2 Stunden bei Raumtemperatur inkubiert, bevor die Vertiefungen mit einer Waschpufferlösung (Wallac) gewaschen wurden. Die Platten wurden dann mit Tribuffered saline (TBS) S, welches 5% BSA enthielt, eine Stunde bei Raumtemperatur blockiert und dann 3 x mit dem Waschpuffer (Wallac) gewaschen, bevor mit dem Reagenzpuffer (Wallac), der den mit Europium markierten monoklonalen Antikörper 3F4 enthielt, 2 Stunden bei Raumtemperatur unter Schütteln inkubiert wurde. Die Platten wurden dann 7 x mit dem Waschpuffer gewaschen, bevor 200 µl/Vertiefung der Verstärkerlösung (Wallac) zugesetzt wurde. Nach 10-minütigem Schütteln bei Raumtemperatur wurde die Fluoreszenz, die von dem mit Europium verbundenen monoklonalen Antikörper 3F4 stammte, in einem Discovery (Canberra Packard) Fluoreszenz-Analysator gemessen. Die Anzahl der Fluoreszenzsignale, die von dem Analysator in der denaturierten Probe gemessen wurden, wurden dann durch die Anzahl der Fluoreszenzsignale geteilt, die von den nativen Proben erhalten wurden. Ein Verhältnis denaturiert/nativ, welches höher war als das Verhältnis erhalten für den Verdünnungspuffer PBS/BSA/Sarkosin zeigte das Vorhandensein von Protease resistenten PrP^{Sc} in der Verdünnungsprobe an (Abb. 2A). Der Sandwich-CDI weist eine 10- bis 30-fache Erhöhung der Empfindlichkeit gegenüber dem herkömmlichen Glutaraldehyd-Vernetzungsverfahren auf.

### 3. Verbessertes konformationsabhängiges Immunoassayverfahren ohne Behandlung mit Proteinase K

Gehirnlysate, die humane Prionen enthielten, wurden in kleiner Menge in humanes Plasma gegeben und in halben log₁₀-Stufen in humanem Plasma verdünnt. Die Verdünnungen wurden dann so behandelt, wie im Beispiel 2 angegeben, nur wurde keine Proteinase K zugegeben (Abb. 2B). Die Empfindlichkeit des Tests wird durch die Sandwich-Konfiguration unter Verwendung der monoklonalen Antikörper aus der Zelllinie DSM ACC 2523 um das 100- bis 300-fache gesteigert gegenüber dem Glutaraldehyd-Vernetzungs-verfahren.

### 4. Inhibition der Antikörperbindung nach Reduktion der Prion-Proteine

Gerenigtes PrP^{Sc}, welches durch die Methode von Bolton (Bolton DC, 1987) aus einem Gehirn eines verstorbenen vCJD-Patienten isoliert wurde, wurde in phosphat-gepufferter Kochsalzlösung welche 2% Sarcosyl und 1% bovines Serumalbumin enthielt, suspendiert, je 1 ml in ein Reaktionsgefäß gegeben, und mit Proteinase K bei einer Enzymkonzentration von 62,5 µg/ml eine Stunde bei 37°C verdaut. Die Reaktion wurde mit dem Fachmann bekannten Proteaseinhibitoren gestoppt und das PrP^{Sc} mittels Zugabe von NaCI zu einer Endkonzentration von 30 Gewichts- pro Volumenprozenten präzipitiert. Die Proben wurden über Nacht bei 4°C stehen gelassen und nach 30-minütiger Zentrifugation bei 16000 g sedimentiert. Das Sediment wurde dann in 50 µl sterilen Wassers, welches dem Fachmann bekannte Proteinaseinhibitoren enthielt, resuspendiert und in Anwesenheit von 4 molarer Guanidinium-Hydrochloridlösung 6 Minuten bei 83°C denaturiert. Parallel hierzu wurden drei gleiche Ansätze unter gleichen Bedingungen denaturiert, aber das PrP^{Sc} wurde hier auch gleichzeitig in Anwesenheit von 3,3 mmol/l Dithiothreitol reduziert. Eine dieser reduzierten Proben wurde 10 Minuten mit 10 mmol/l Jodazetamid behandelt, um die Thiol-Reste irreversibel zu methylieren und ihre Re-oxidation zu verhindern. Die zweite reduzierte Probe wurde mittels 0,2 mmol/l oxidierten Gluthathions 2 Stunden bei Raumtemperatur re-oxidiert. Den anderen Versuchsansätzenwurde das gleiche Volumken an Puffer ohne Gluthathion zugesetzt, und sie wurden ebenfalls 2 Stunden bei Raumtemperatur stehen gelassen.
Danach wurden jeweils 3 mal 200 µl einer Probe in eine Vertiefung einer Mikrotiterplatte, welche mit Glutaraldehyd wie in Beispiel 2 prä-aktiviert war, überführt und 2 Stunden lang unter Schütteln inkubiert. Nach Blockieren und Waschen der Mikrotiterplatten-Vertiefungen wurde monoklonaler Antikörper DSM ACC 2522, DSM ACC 2523 oder DSM ACC 2523 zugegeben. Nach 1,5-stündiger Inkubation bei Raumtemperatur wurde der Antikörper durch siebenmaliges Waschen der Mikrotiterplatte entfernt. Ein Zweitantikörper spezifisch für murine Immunglobuline, welcher mittels chemischer Kopplung mit einem Europium-Chelatkomplex markiert war, wurde dann hinzugegeben und ebenfalls 1,5 Stunden bei Raumtemperatur unter Schütteln inkubiert. Nach Entfernung des Zweitantikörpers und siebenmaligem Waschen wurde das Europium von dem gebundenen Zweitantikörper aus dem Chelatkomplex verdrängt mittels geeigneten Enhance-Puffers der Fa. Wallac (Turku, Finnland). Das Europium-Fluoreszenzsignal wurde dann in einem Discovery-Fluoreszenzmessgerät (Fa. Canberra Packard, Darmstadt) analysiert. Die nicht reduzierten PrP^{Sc}-Moleküle erzeugten ein etwa 30-fach höheres Signal als die reduzierten PrP^{Sc}-Moleküle (Abbildung 3). Dies bedeutet, dass für eine effiziente Bindung der Antikörper DSM ACC 2522, DSM AC 2523, DSM ACC 2524 an das humane Prion-Protein eine Disulfidbrücke im Prion Protein bestehen muss. Die Erkennung der einmal reduzierten PrP^{Sc}-Moleküle kann nach Wiederherstellung der Disulfid-Brücke zum größten Teil wieder hergestellt werden. Dies ist in Abbildung 3 ebenfalls gezeigt: nach Oxidation der reduzierten PrP^{Sc}-Moleküle mittels oxidierten Gluthathions oder mittels Luftsauerstoffs, die jeweils als Oxidationsmittel verwendet wurden, erreichte die Bindung des Europium-markierten Antikörpers bis zu 60% der Werte der nichtreduzierten PrP^{Sc}-Probe.

### 4. Inhibition der Antikörperbindung nach Reduktion der Prion-Proteine

Gereinigtes PrP^{Sc}, welches durch die Methode von Bolton (Bolton DC, 1987) aus einem Gehirn eines verstorbenen vCJD-Patienten isoliert wurde, wurde in phosphat-gepufferter Kochsalzlösung welche 2% Sarcosyl und 1% bovines Serumalbumin enthielt, suspendiert, je 1 ml in ein Reaktionsgefäß gegeben, und mit Proteinase K bei einer Enzymkonzentration von 62,5 µg/ml eine Stunde bei 37°C verdaut. Die Reaktion wurde mit dem Fachmann bekannten Proteaseinhibitoren gestoppt und das PrP^{Sc} mittels Zugabe von NaCI zu einer Endkonzentration von 30 Gewichts- pro Volumenprozenten präzipitiert. Die Proben wurden über Nacht bei 4°C stehen gelassen und nach 30-minütiger Zentrifugation bei 16000 g sedimentiert. Das Sediment wurde dann in 50 µl sterilen Wassers, welches dem Fachmann bekannte Proteinaseinhibitoren enthielt, resuspendiert und in Anwesenheit von 4 molarer Guanidinium-Hydrochloridlösung 6 Minuten bei 83°C denaturiert. Parallel hierzu wurden drei gleiche Ansätze unter gleichen Bedingungen denaturiert, aber das PrP^{Sc} wurde hier auch gleichzeitig in Anwesenheit von 3,3 mmol/l Dithiothreitol reduziert. Eine dieser reduzierten Proben wurde 10 Minuten mit 10 mmol/l Jodazetamid behandelt, um die Thiol-Reste irreversibel zu methylieren und ihre Re-oxidation zu verhindern. Die zweite reduzierte Probe wurde mittels 0,2 mmol/l oxidierten Gluthathions 2 Stunden bei Raumtemperatur re-oxidiert. Den anderen Versuchsansätzenwurde das gleiche Volumken an Puffer ohne Gluthathion zugesetzt, und sie wurden ebenfalls 2 Stunden bei Raumtemperatur stehen gelassen.
Danach wurden jeweils 3 mal 200 µl einer Probe in eine Vertiefung einer Mikrotiterplatte, welche mit Glutaraldehyd wie in Beispiel 2 prä-aktiviert war, überführt und 2 Stunden lang unter Schütteln inkubiert. Nach Blockieren und Waschen der Mikrotiterplatten-Vertiefungen wurde monoklonaler Antikörper DSM ACC 2522, DSM ACC 2523 oder DSM ACC 2523 zugegeben. Nach 1,5-stündiger Inkubation bei Raumtemperatur wurde der Antikörper durch siebenmaliges Waschen der Mikrotiterplatte entfernt. Ein Zweitantikörper spezifisch für murine Immunglobuline, welcher mittels chemischer Kopplung mit einem Europium-Chelatkomplex markiert war, wurde dann hinzugegeben und ebenfalls 1,5 Stunden bei Raumtemperatur unter Schütteln inkubiert. Nach Entfernung des Zweitantikörpers und siebenmaligem Waschen wurde das Europium von dem gebundenen Zweitantikörper aus dem Chelatkomplex verdrängt mittels geeigneten Enhance-Puffers der Fa. Wallac (Turku, Finnland). Das Europium-Fluoreszenzsignal wurde dann in einem Discovery-Fluoreszenzmessgerät (Fa. Canberra Packard, Darmstadt) analysiert. Die nicht reduzierten PrP^{Sc}-Moleküle erzeugten ein etwa 30-fach höheres Signal als die reduzierten PrP^{Sc}-Moleküle (Abbildung 3). Dies bedeutet, dass für eine effiziente Bindung der Antikörper DSM ACC 2522, DSM AC 2523, DSM ACC 2524 an das humane Prion-Protein eine Disulfidbrücke im Prion Protein bestehen muss. Die Erkennung der einmal reduzierten PrP^{Sc}-Moleküle kann nach Wiederherstellung der Disulfid-Brücke zum größten Teil wieder hergestellt werden. Dies ist in Abbildung 3 ebenfalls gezeigt: nach Oxidation der reduzierten PrP^{Sc}-Moleküle mittels oxidierten Gluthathions oder mittels Luftsauerstoffs, die jeweils als Oxidationsmittel verwendet wurden, erreichte die Bindung des Europium-markierten Antikörpers bis zu 60% der Werte der nichtreduzierten PrP^{Sc}-Probe.

### Beschreibung der Abbildungen:

Abbildung 1: Bindung der erfindungsgemäßen Antikörper an humanes Prionprotein. Hirnhomogenate unterschiedlicher Spezies wurden im SD-Polyacrylamid-Gel aufgetrennt und nach Elektrotransfer auf Nylonmembranen transferiert. Der anschließende Westernblot wurde mit dem monoklonalen Antikörper 6H4 (Fa. Prionics AG, Schweiz, obere Abbildung) und parallel mit dem erfindungsgemäßen Antikörper der Hybridomazellinie DSM ACC 2522 (untere Abbildung) durchgeführt. Gebundene Antikörper wurden mittels Phosphatase-gekoppeltem Zweitantikörper nachgewiesen. Spur 1: Molekulargewichtsstandard; Spur 2: Rattenhirnhomogenat; Spur 3: Menschenhirnhomogenat; Spur 4: Hirnhomogenat der afrikanischen Grünen Meerkatze; Spur 5: Hirnhomogenat des syrischen Goldhamsters; Spur 6: Rinderhirnhomogenat.

Abbildung 3: Bindung des monoklonalen Antikörpers an humanes Prionprotein. Humanes, pathogenes Prionprotein, PrP^{Sc}, wurde denaturiert und vor der Immobilisierung an einer Microtiterplatte entweder mit DTT reduziert und mit Jodacetamide methyliert, oder DTT reduziert und mit Glutathione oxidiert, oder mit DTT reduziert und mit Luftsauerstoff (O2) oxidiert. Die immobiliserten Prionproteine wurden dann mit dem erfindungsgemäßen, monoklonalen Antikörper der Zelllinie DMS ACC 2523 inkubiert und die gebundenen Antikörper mit einen Fluoreszenzmarkierten Zweitantikörper nachgewiesen. TRF: gemessene Fluoreszenereignisse (time-resolved fluorescence). Reduzierung der Disulfidbrücke im Prionprotein führt zum Verlust des Fluoreszenzsignals und damit der Antikörperbindung.

### Referenz Liste

Barry RA, et al. (1986). **Monoclonal antibodies to the cellular and scrapie prion proteins.** J Infect Dis *154,* 518-521.

Bolton DC, et al. (1987). **Isolation and structural studies of the intact scrapie agent protein.** Arch Biochem Biophys *258,* 579-590.

Harmeyer S, et al. (1998). **Synthetic peptide vaccines yield monoclonal antibodies to cellular and pathological prion proteins of ruminants.** J Gen Virol *79*, 937-945.

Krasemann S, et al. (1999). **Generation of monoclonal antibodies against prion proteins with an unconventional nucleic acid-based immunization strategy.** J Biotechnol *73*, 119-129.

Prusiner SB, et al. (1982). **Further purification and characterization of scrapie prions.** Biochemistry *21,* 6942-6950.

Prusiner SB, et al. (1984). **Purification and structural studies of a major scrapie prion protein**. Cell *38*, 127-134.

Prusiner SB. (1998). **Prions.** Proc Natl Acad Sci U S A *95,* 13363-13383.

Smales CM, et al. (2002). **Protein modification during anti-viral heat-treatment bioprocessing of factor VIII concentrates, factor IX concentrates, and model proteins in the presence of sucrose.** Biotechnol Bioeng 77, 37-48.

Zanusso G, et al. (1998). **Prion protein expression in different species: analysis with a panel of new mAbs.** Proc Natl Acad Sci U S A 95, 8812-8816.

## Patentansprüche

1. Konformationelles Epitop, das für humanes Prionprotein spezifisch ist und welches nur unter oxidierenden Bedingungen im Prionprotein vorhanden ist, **dadurch gekennzeichnet, dass** es von mindestens einem der Antikörper erkannt wird, die von einer der Hybridoma-Zelllinien DSM ACC 2522, DSM ACC 2523 oder DSM ACC 2524 gebildet werden.

2. Monoklonaler Antikörper, **dadurch gekennzeichnet, dass** er spezifisch an das Epitop gemäß Anspruch 1 bindet.

3. Monoklonaler Antikörper nach Anspruch 2, **dadurch gekennzeichnet, dass** er nicht an Prionprotein der afrikanischen grünen Meerkatze, des Rindes, des syrischen Goldhamsters, der Maus oder der Ratte bindet.

4. Monoklonaler Antikörper nach Anspruch 3, **dadurch gekennzeichnet, dass** er von einer der Hybridoma-Zelllinien DSM ACC 2522, DSM ACC 2523 oder DSM ACC 2524 gebildet wird.

5. Verfahren zum Nachweis von pathogenen Prionen humanen Ursprungs, **dadurch gekennzeichnet, dass** ein monoklonaler Antikörper nach den Ansprüchen 1 bis 4 in dem Nachweisverfahren eingesetzt wird.

6. Konformationsabhängiges Immunoassayverfahren zum Nachweis pathogener Prionproteine in der Probe einer Körperflüssigkeit menschlichen oder tierischen Ursprungs, enthaltend ein PrP-Protein, das eine erste natürliche, nichtpathologische Konformation, PrP^{c}, und eine zweite, pathologische Konformation, PrP^{Sc} genannt, einnimmt, wobei die genannten Proteine sich in ihrer Bindungsaffinität an monoklonale Antikörper gemäß den Ansprüchen 2 bis 4 unterscheiden, wobei das Verfahren folgende Stufen umfasst:
a) Versetzen einer ersten Portion der Probe mit einem der vorstehend genannten monoklonalen Antikörper, der an einen festen Träger fixiert ist und eine höhere Affinität für die erste Prionprotein-Konformation aufweist, sowie die Bestimmung dieser ersten Konzentration;
b) Behandlung der zweiten Portion der Probe zur Erhöhung der Bindungsaffinität der zweiten Konformation des Prionproteins an dem monoklonalen Antikörper;
c) Versetzen der behandelten zweiten Portion der zu untersuchenden Probe mit dem monoklonalen Antikörper, um die zweite Konzentration zu ermitteln;
d) Vergleichen der ersten Prionprotein-Konzentration mit der zweiten Prionprotein-Konzentration, um die Anwesenheit der pathogenen Prionprotein-Konformation zu ermitteln.

7. Immunoassayverfahren nach den Ansprüchen 5 und 6, **dadurch gekennzeichnet, dass** die Probe mit einem proteolytischen Enzym vorbehandelt wird.

8. Immunoassayverfahren nach den Ansprüchen 5 bis 7, **dadurch gekennzeichnet, dass** das proteolytische Enzym aus der Gruppe umfassend Proteinase K oder Dispase ausgewählt ist.

9. Immunoassayverfahren nach den Ansprüchen 5 bis 8, **dadurch gekennzeichnet, dass** die zu untersuchende Probe einer Behandlung unterworfen wird, bei der sie zur Änderung der Konformation erhöhten Temperaturen, Druck oder chemischen Reagenzien ausgesetzt sind, durch die wenigstens 2% eines etwa anwesenden Prionproteins in eine an den monoklonalen Antikörper bindende Form umgewandelt wird.

10. Immunoassayverfahren nach den Ansprüchen 4 bis 9, **dadurch gekennzeichnet, dass** die Menge der an dem festen Träger fixierten Prionproteine durch Anwendung eines zweiten markierten monoklonalen Antikörpers gemessen wird.

11. Immunoassayverfahren nach Anspruch 10, **dadurch gekennzeichnet, dass** als zweiter markierter monoklonaler Antikörper der monoklonale Antikörper 3F4, welcher von den Unter der Nummer ATCC-HB 9222 hinterlegten Hybridomzellen produziert wird, verwendet wird.

12. Immunoassayverfahren nach den Ansprüchen 10 und 11, **dadurch gekennzeichnet, dass** der monoklonale Antikörper mit einer radioaktiven Gruppe, einer enzymatischen Gruppe oder einer fluoreszierenden Gruppe markiert ist.

## Claims

1. Conformational epitope specific for human prion protein and only present in prion protein under oxidizing conditions, **characterized in that** it is recognized by at least one of the antibodies formed by one of the hybridoma cell lines DSM ACC 2522, DSM ACC 2523 or DSM ACC 2524.

2. Monoclonal antibody, **characterized in that** it binds specifically to the epitope of Claim 1.

3. Monoclonal antibody according to Claim 2, **characterized in that** it does not bind to prion protein from the African vervet monkey, the cow, the Syrian golden hamster, the mouse or the rat.

4. Monoclonal antibody according to Claim 3, **characterized in that** it is formed by one of the hybridoma cell lines DSM ACC 2522, DSM ACC 2523 and DSM ACC 2524.

5. Method for detecting pathogenic prions of human origin, **characterized in that** a monoclonal antibody according to Claims 1 to 4 is used in the detection method.

6. Conformation-dependent immunoassay method for detecting pathogenic prion proteins in the sample of a body fluid of human or animal origin, which sample contains a PrP protein which adopts a first, natural, nonpathological conformation, i.e. PrP^{c}, and a second, pathological conformation, i.e. PrP^{Sc}, with said proteins differing in their binding affinity for monoclonal antibodies according to Claims 2 to 4, with the method comprising the following steps:
a) adding one of the abovementioned monoclonal antibodies, which is fixed to a solid support and which exhibits a higher affinity for the first prion protein conformation, to a first portion of the sample, and determining this first concentration;
b) treating the second portion of the sample in order to increase the binding affinity of the second conformation of the prion protein for the monoclonal antibody;
c) adding the monoclonal antibody to the treated second portion of the sample to be investigated, in order to determine the second concentration;
d) comparing the first prion protein concentration with the second prion protein concentration in order to ascertain the presence of the pathogenic prion protein conformation.

7. Immunoassay method according to Claims 5 and 6, **characterized in that** the sample is pretreated with a proteolytic enzyme.

8. Immunoassay method according to Claims 5 to 7, **characterized in that** the proteolytic enzyme is selected from the group comprising proteinase K or dispase.

9. Immunoassay method according to Claims 5 to 8, **characterized in that** the sample to be investigated is subjected to a treatment in which, for the purpose of change in the conformation, it is exposed to elevated temperatures, pressure or chemical reagents, which convert at least 2% of a prion protein which may possibly be present into a form which binds to the monoclonal antibody.

10. Immunoassay method according to Claims 4 to 9, **characterized in that** the quantity of the prion proteins attached to the solid support is measured by using a second, labelled monoclonal antibody.

11. Immunoassay method according to Claim 10, **characterized in that** the monoclonal antibody 3F4, which is produced by the hybridoma cells deposited under the number ATCC-HB 9222, is used as the second, labelled monoclonal antibody.

12. Immunoassay method according to Claims 10 and 11, **characterized in that** the monoclonal antibody is labelled with a radioactive group, an enzymatic group or a fluorescent group.

## Revendications

1. Epitope conformationnel qui est spécifique à la protéine prion humaine et qui est présent dans la protéine prion uniquement en conditions oxydantes, **caractérisé en ce qu'**il est reconnu par au moins l'un des anticorps qui sont formés par l'une des lignées cellulaires d'hybridomes DSM ACC 2522, DSM ACC 2523 ou DSM ACC 2524.

2. Anticorps monoclonal, **caractérisé en ce qu'**il se lie de manière spécifique à l'épitope selon la revendication 1.

3. Anticorps monoclonal selon la revendication 2, **caractérisé en ce qu'**il ne se lie pas à la protéine prion du singe vert africain, des bovins, du hamster doré de Syrie, de la souris ou du rat.

4. Anticorps monoclonal selon la revendication 3, **caractérisé en ce qu'**il est formé par l'une des lignées cellulaires d'hybridomes DSM ACC 2522, DSM ACC 2523 ou DSM ACC 2524.

5. Méthode pour la détection de prions pathogènes d'origine humaine, **caractérisée en ce qu'**un anticorps monoclonal selon les revendications 1 à 4 est utilisé dans la méthode de détection.

6. Méthode d'immunodosage en fonction de la conformation pour détecter des protéines prions pathogènes dans un échantillon de fluide corporel d'origine humaine ou animale, contenant une protéine PrP qui prend une première conformation naturelle non pathologique, désignée par PrP^{c}, et une deuxième conformation pathologique, désignée par PrP^{Sc}, lesdites protéines se différenciant par leur affinité de liaison à des anticorps monoclonaux selon les revendications 2 à 4, la méthode comprenant les étapes consistant à :
a) Mélanger une première portion de l'échantillon avec l'un des anticorps monoclonaux précités, qui est fixé sur un support solide et qui présente une plus grande affinité pour la première conformation de la protéine prion, ainsi que déterminer cette première concentration ;
b) Traiter la deuxième portion de l'échantillon afin d'augmenter l'affinité de liaison de la deuxième conformation de la protéine prion à l'anticorps monoclonal ;
c) Mélanger la deuxième portion traitée de l'échantillon à examiner avec l'anticorps monoclonal afin de déterminer la deuxième concentration ;
d) Comparer la première concentration en protéine prion à la deuxième concentration en protéine prion afin de déterminer la présence de la conformation pathogène de la protéine prion.

7. Méthode d'immunodosage selon les revendications 5 et 6, **caractérisée en ce que** l'échantillon est prétraité avec une enzyme protéolytique.

8. Méthode d'immunodosage selon les revendications 5 à 7, **caractérisée en ce que** l'enzyme protéolytique est choisie dans le groupe comprenant la protéinase K ou la dispase.

9. Méthode d'immunodosage selon les revendications 5 à 8, **caractérisée en ce que** l'échantillon à examiner est soumis à un traitement dans lequel, pour modifier la conformation, il est exposé à des températures élevées, à une pression ou à des réactifs chimiques qui font que 2% au moins d'une protéine prion éventuellement présente sont convertis en une forme qui se lie à l'anticorps monoclonal.

10. Méthode d'immunodosage selon les revendications 4 à 9, **caractérisée en ce que** la quantité des protéines prions fixées sur le support solide est mesurée en utilisant un deuxième anticorps monoclonal marqué.

11. Méthode d'immunodosage selon la revendication 10, **caractérisée en ce que** le deuxième anticorps monoclonal marqué utilisé est l'anticorps monoclonal 3F4, qui est produit par les cellules d'hybridome déposées sous le numéro ATCC HB9222.

12. Méthode d'immunodosage selon les revendications 10 et 11, **caractérisée en ce que** l'anticorps monoclonal est marqué au moyen d'un groupe radioactif, d'un groupe enzymatique ou d'un groupe fluorescent.
